Europäisches Patentamt

European Patent Office

Office européen des brevets

(19)

(11) Veröffentlichungsnummer: **0 120 182**
**B1**

## (12) EUROPÄISCHE PATENTSCHRIFT

(45) Veröffentlichungstag der Patentschrift:
25.09.85

(51) Int. Cl.⁴: **C 07 C 149/243**

(21) Anmeldenummer: 84100179.5

(22) Anmeldetag: **10.01.84**

(54) Verfahren zur Gewinnung von D,L-Homocystin.

(30) Priorität: **18.03.83 DE 3309761**

(43) Veröffentlichungstag der Anmeldung:
**03.10.84 Patentblatt 84/40**

(45) Bekanntmachung des Hinweises auf die Patenterteilung:
**25.09.85 Patentblatt 85/39**

(84) Benannte Vertragsstaaten:
**AT BE CH FR GB IT LI LU NL SE**

(56) Entgegenhaltungen:
**US - A - 2 406 362**

**JOURNAL OF AGRICULTURAL AND FOOD CHEMISTRY, Band 26, Nr. 6, November/Dezember 1978, Seiten 1406-1409, American Chemical Society; S.H. LIPTON: "Peroxide oxidation products of homocystine and lanthionine"**

(73) Patentinhaber: **Degussa Aktiengesellschaft, Weissfrauenstrasse 9, D-6000 Frankfurt am Main 1 (DE)**

(72) Erfinder: **Karrenbauer, Michael, Dr. Dipl.-Chem., Lindenstrasse 10, D-6458 Rodenbach (DE)**
Erfinder: **Kleemann, Axel, Dr. Dipl.-Chem., Greifenhagenstrasse 25, D-6450 Hanau 9 (DE)**
Erfinder: **Lüssling, Theodor, Dr. Dipl.-Chem., Zum Purren 5, D-7750 Konstanz Litzelstetten (DE)**
Erfinder: **Schäfer, Fritz, Dr. Dipl.-Chem., Uhlandstrasse 25, D-7750 Konstanz (DE)**

## Beschreibung

Gegenstand der Erfindung ist ein Verfahren zur Gewinnung von D,L-Homocystin durch Oxidation des Dinatriumsalzes von D,L-Homocystein.

D,L-Homocystin ist als Zusatz bei der Herstellung von Heimtiernahrung von Interesse.

Es ist zwar bekannt, Mercaptane mit Wasserstoffperoxid zu den entsprechenden Disulfiden zu oxidieren. Im Falle des D,L-Homocysteins werden jedoch nur bei Einhaltung ganz bestimmter Bedingungen hohe Ausbeuten an dem gewünschten D,L-Homocystin erhalten.

Das erfindungsgemässe Verfahren ist dadurch gekennzeichnet, dass man eine wässerige Lösung des Dinatriumsalzes von D,L-Homocystein mit einer Konzentration zwischen 0,4 und 1,6 Mol/l und einem Anfangs-pH zwischen 7,0 und 8,0 unter kräftigem Rühren mit der mindestens äquivalenten Menge einer wässerigen Wasserstoffperoxidlösung versetzt und nach beendeter Oxidation den pH auf etwa 5,3 einstellt.

Unter diesen Reaktionsbedingungen wird überraschenderweise das gewünschte D,L-Homocystin in Ausbeuten von über 80% erhalten. Setzt man dagegen die wässerige Lösung des Dinatriumsalzes von D,L-Homocystein mit einer Konzentration von weniger als 0,4 Mol/l oder mehr als 1,6 Mol/l oder mit einem Anfangs-pH von weniger als 7,0 oder mehr als 8,0 ein, werden deutlich geringere Ausbeuten erhalten.

Die als Ausgangsmaterial dienenden wässerigen Lösungen des Dinatriumsalzes von D,L-Homocystein können in bekannter Weise durch Entmethylierung von D,L-Methionin mittels Natrium in flüssigem Ammoniak, anschliessendes Abdampfen des Ammoniaks und Aufnehmen des verbleibenden Rückstandes in einer geeigneten Menge Wasser hergestellt werden.

Die Einstellung des Anfangs-pH auf einen Wert zwischen 7,0 und 8,0 erfolgt zweckmässigerweise mit einer wässerigen Mineralsäure, vorzugsweise Salzsäure.

Die wässerige Wasserstoffperoxidlösung wird zweckmässigerweise in der äquivalenten Menge eingesetzt. Die Anwendung eines geringen Überschusses bis zu 5% stört jedoch nicht. Vorzugsweise werden wässerige Wasserstoffperoxidlösungen mit einer Konzentration zwischen 20 und 60 Gewichtsprozent eingesetzt. Da die Oxidationsreaktion stark exotherm ist, empfiehlt es sich, die Wasserstoffperoxidlösung langsam tropfenweise zuzugeben und das Reaktionsgemisch während der Zugabe zu kühlen. Die Oxidationsreaktion ist im allgemeinen beendet, wenn das gesamte Wasserstoffperoxid zugegeben ist.

Nach beendeter Oxidation wird der pH des Reaktionsgemisches, zweckmässigerweise mit einer wässerigen Mineralsäure, vorzugsweise Salzsäure, auf etwa 5,3 eingestellt. Es ist vorteilhaft, wenn die Einstellung des pH bei erhöhter Temperatur, beispielsweise 50° C, vorgenommen wird und anschliessend das Reaktionsgemisch unter Rühren langsam, beispielsweise im Verlauf einer Stunde, auf Raumtemperatur abgekühlt wird. In einer besonders vorteilhaften kristallinen Form fällt das D,L-Homocystin dann an, wenn die Rührgeschwindigkeit während der Ausfällung so bemessen wird, dass das D,L-Homocystin gerade noch in Suspension gehalten wird. Es kann dann besonders leicht durch Filtration oder Zentrifugation abgetrennt werden.

Die Erfindung wird durch die nachfolgenden Beispiele und Vergleichsversuche näher erläutert. Prozentangaben bedeuten, wenn nicht anders angegeben, Gewichtsprozente.

### Beispiel 1

21,6 g durch Entmethylierung von D,L-Methionin hergestelltes Dinatriumsalz von D,L-Homocystein werden in Wasser gelöst und mit Salzsäure versetzt, so dass eine 0,4molare Lösung mit einem pH von 7,0 entsteht. Diese Lösung wird bei indirekter Kühlung mit Eis unter kräftigem Rühren tropfenweise mit 6 ml einer 50%igen Wasserstoffperoxidlösung versetzt. Anschliessend wird das Reaktionsgemisch auf 50° C erwärmt und unter Rühren mit 10%iger Salzsäure auf einen pH von 5,3 eingestellt. Unter Rühren wird innerhalb einer Stunde auf Raumtemperatur abgekühlt. Das ausgefällte D,L-Homocystin wird abgesaugt, mit 100 ml Wasser von 80° C nachgewaschen und im Vakuumtrockenschrank bis zur Gewichtskonstanz getrocknet. Die Ausbeute an D,L-Homocystin beträgt 14,7 g, entsprechend 90,5% der Theorie.

### Beispiel 2

Das Beispiel 1 wird wiederholt mit dem Unterschied, dass das Dinatriumsalz von D,L-Homocystein mit soviel Wasser und Salzsäure versetzt wird, dass eine 0,4molare Lösung mit einem pH von 8,0 entsteht. Die Ausbeute an D,L-Homocystin beträgt 13,3 g, entsprechend 81,7% der Theorie.

### Vergleichsversuch 1

Das Beispiel 1 wird wiederholt mit dem Unterschied, dass das Dinatriumsalz von D,L-Homocystein mit soviel Wasser und Salzsäure versetzt wird, dass eine 0,4 molare Lösung mit einem pH von 10,0 entsteht. Die Ausbeute an D,L-Homocystin beträgt 10,7 g, entsprechend 65,7% der Theorie.

### Beispiel 3

43,2 g durch Entmethylierung von D,L-Methionin hergestelltes Dinatriumsalz von D,L-Homocystein werden in Wasser gelöst und mit Salzsäure versetzt, so dass eine 0,8molare Lösung mit einem pH von 7,0 entsteht. Diese Lösung wird bei indirekter Kühlung mit Eis unter kräftigem Rühren tropfenweise mit 12 ml einer 50%igen Wasserstoffperoxidlösung versetzt und weiterbehandelt wie im Beispiel 1. Die Ausbeute an D,L-Homocystin beträgt 29,3 g, entsprechend 90,1% der Theorie.

### Beispiel 4

Das Beispiel 3 wird wiederholt mit dem Unterschied, dass das Dinatriumsalz von D,L-Homocy- stein mit soviel Wasser und Salzsäure versetzt wird, dass eine 0,8molare Lösung mit einem pH von 8,0 entsteht. Die Ausbeute an D,L-Homocystin beträgt 30,2 g, entsprechend 93,0% der Theorie.

*Vergleichsversuch 2*

Das Beispiel 3 wird wiederholt mit dem Unterschied, dass das Dinatriumsalz von D,L-Homocystein mit soviel Wasser und Salzsäure versetzt wird, dass eine 0,8molare Lösung mit einem pH von 10,0 entsteht. Die Ausbeute an D,L-Homocystin beträgt 23,1 g, entsprechend 71,0% der Theorie.

*Beispiel 5*

86,4 g durch Entmethylierung von D,L-Methionin hergestelltes Dinatriumsalz von D,L-Homocystein werden in Wasser gelöst und mit Salzsäure versetzt, so dass eine 1,6molare Lösung mit einem pH von 7,0 entsteht. Diese Lösung wird bei indirekter Kühlung mit Eis unter kräftigem Rühren tropfenweise mit 24 ml einer 50%igen Wasserstoffperoxidlösung versetzt und weiterbehandelt wie im Beispiel 1. Die Ausbeute an D,L-Homocystin beträgt 58,5 g, entsprechend 90% der Theorie.

*Beispiel 6*

Das Beispiel 5 wird wiederholt mit dem Unterschied, dass das Dinatriumsalz von D,L-Homocystein mit soviel Wasser und Salzsäure versetzt wird, dass eine 1,6molare Lösung mit einem pH von 8,0 entsteht. Die Ausbeute an D,L-Homocystin beträgt 60,0 g, entsprechend 92,3% der Theorie.

*Vergleichsversuche 3 bis 5*

Lösungen gleicher Konzentration wie im Beispiel 5, aber mit Anfangs-pH-Werten von 10,0, 11,0 und 12,0, werden wie im Beispiel 5 behandelt. Die Ergebnisse sind in der nachfolgenden Tabelle zusammengestellt:

| Anfangs-pH | Ausbeute |
|------------|----------|
| 10,0 | 47,5 g (73,0% der Theorie) |
| 11,0 | 44,5 g (68,5% der Theorie) |
| 12,0 | 34,5 g (53,0% der Theorie) |

**Patentansprüche**

1. Verfahren zur Gewinnung von D,L-Homocystin durch Oxidation des Dinatriumsalzes von D,L-Homocystein, dadurch gekennzeichnet, dass man eine wässerige Lösung des Dinatriumsalzes von D,L-Homocystein mit einer Konzentration zwischen 0,4 und I,6 Mol/l und einem Anfangs-pH zwischen 7,0 und 8,0 unter kräftigem Rühren mit der mindestens äquivalenten Menge einer wässerigen Wasserstoffperoxidlösung versetzt und nach beendeter Oxidation den pH auf etwa 5,3 einstellt.

2. Verfahren nach Anspruch 1, dadurch gekennzeichnet, dass man eine wässerige Wasserstoffperoxidlösung mit einer Konzentration zwischen 20 und 60 Gewichtsprozent einsetzt.

**Claims**

1. A process for the production of D,L-homocystine by oxidizing the disodium salt of D,L-homysteine, characterized in that an aqueous solution of the disodium salt of D,L-homocysteine with a concentration of from 0.4 to 1.6 mol/l and an initial pH of from 7.0 to 8.0 is treated wit vigorous stirring with at least an equivalent quantity of an aqueous hydrogen peroxide solution and when oxidation is complete the pH is adjusted to about 5.3.

2. A process according to Claim 1, characterized in that an aqueous hydrogen peroxide solution with a concentration of from 20 to 60% by weight is used.

**Revendications**

1. Procédé pour l'obtention de D,L-homocystine par oxydation du sel disodique de D,L-homocystéine, caractérisé en ce que l'on ajoute à une solution aqueuse du sel disodique de la D,L-homocystéine ayant une concentration de 0,4 à 1,6 mole/l et un pH initial de 7 à 8, en agitant énergiquement, une quantité au moins équivalente d'eau oxygénée, et ajuste le pH à environ 5,3 quand l'oxydation est achevée.

2. Procédé suivant la revendication 1, caractérisé en ce que l'on utilise de l'eau oxygénée à une concentration qui se situe entre 20 et 60% en poids.